(19) **Europäisches Patentamt / European Patent Office / Office européen des brevets**

(11) **EP 1 348 425 B1**

(12) **EUROPEAN PATENT SPECIFICATION**

(45) Date of publication and mention
of the grant of the patent:
**02.07.2008 Bulletin 2008/27**

(51) Int Cl.:
*A61K 8/02* (2006.01)          *A61L 15/58* (2006.01)
*A61L 24/04* (2006.01)

(21) Application number: **03447063.3**

(22) Date of filing: **27.03.2003**

(54) **Hydrogel adhesives for use on fiber-populated surfaces**

Hydrogel-Klebstoffe zur Verwendung auf Fasern besetzten Oberflächen

Adhesifs à base d'hydrogel pour l'utilisation sur des surfaces fibreuses

(84) Designated Contracting States:
**AT BE BG CH CY CZ DE DK EE ES FI FR GB GR HU IE IT LI LU MC NL PT RO SE SI SK TR**

(30) Priority: **29.03.2002 EP 02447052**

(43) Date of publication of application:
**01.10.2003 Bulletin 2003/40**

(60) Divisional application:
**08150984.6 / 1 925 324**

(73) Proprietor: **THE PROCTER & GAMBLE COMPANY**
**Cincinnati, Ohio 45202 (US)**

(72) Inventors:
• **Cinelli, Fabio**
**65125 Pescara (IT)**

• **Coles, Peter**
**Hamilton, OH 45011 (US)**
• **Goldman, Stephen Allen**
**65013 Citta Sant'Angelo (Pescara) (IT)**
• **Romano, Mario**
**65125 Pescara (IT)**

(74) Representative: **Clemo, Nicholas Graham et al**
**Procter & Gamble Technical Centres Limited**
**Patent Department**
**Rusham Park,**
**Whitehall Lane**
**Egham, Surrey TW20 9NW (GB)**

(56) References cited:
**EP-A- 1 026 219          EP-A- 1 245 240**
**WO-A-00/07636          US-A1- 2002 026 005**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Field of the Invention

The present invention relates to hydrogel adhesives for attachment to fibers populated surfaces in particular to Personal Care or Surface Care products containing such hydrogels.

### Background of the Invention

While the use of hydrogel body adhesives in the of medical field, such as skin electrodes, transdermal drug delivery and wound healing has been known for several years, hydrogel body adhesives have been disclosed more recently for use in consumer products such as absorbent articles and human waste-management articles; representative of the latter disclosures are EP 1 025 823 EP 1 025 866, WO00/07636, EP1026219 and US 2002/026005 where certain needs specific for such consumer products waste-management products, are addressed, including secure attachment, painless removal and stability of adhesion in presence of excess moisture.

It has now been surprisingly found that the above drawbacks will be substantially alleviated by the use of certain hydrogel adhesives with rheology characteristics as defined hereinafter.

Finally it has been found that the benefits of the use and of the hydrogels herein can be applied to surfaces populated with fibers, and thus the present invention also applies to the use of hydrogel with selected characteristics, for surface care articles.

### Summary of the Invention

In a first embodiment herein, the present invention is directed to the use, for adhesion on fiber-populated surfaces, of hydrogel adhesives comprising 10-60 wt% of a cross-linked hydrophilic polymer, 5-80 wt% of a water-soluble nonionic humectant and 10-85wt% water, said adhesive having a viscous modules at a temperature of 25°C, $G'_{25}$, selected such that $G'_{25}$ (1rad/sec) is less than 1000Pa, preferably from 100 to 700 Pa.

In a second embodiment herein, the present invention provides surface-care articles capable of adhering to fiber-populated surface, said surface care articles comprising a hydrogel adhesive as defined in the first embodiment. The hydrogel adhesives herein preferably have the ratio of $G''_{25}$ (1rad/sec)/$G'_{25}$ (1rad/sec) is in the range of 0.15 to 0.65 preferably 0.15 to 0.35 and the peel strength force on dry skin is in the range than 0.3 to 3N/cm preferably 0.3 to 2N/cm.

### Detailed Description

In a first embodiment herein, the present invention is directed to the use, for adhesion on fiber-populated surfaces, of hydrogel adhesives comprising 10-60 wt% of a cross-linked hydrophilic polymer, 5-80 wt% of a water-soluble nonionic humectant and 10-85 wt% water. The polymerization of the monomers preferably takes place in presence of the nonionic humectant and water and cross-linking creates a 3-dimensional matrix for the polymer, also referred to as gel form and hydrogel, as will be described in more detail hereinafter.
A critical factor of the hydrogels herein is rheology, is particularly the elastic behavior.

### Rheology

The viscous behavior of an adhesive can be interpreted to represent an indication of the ability of the adhesive to quickly attach and securely adhere to a particular surface. The elastic behavior can be interpreted as an indication of the "hardness" behavior of the adhesive. Its value is also important for good initial attachment. Their combination is believed to be an indicator of the required force upon removal. The relation between elastic and viscous modulus is considered to be an indication on which fraction of the removal energy will be dissipated within the adhesive and which fraction is available to trigger the actual removal.

While not being bound by theory, it is believed that for hydrogels applied to skin, the rheological properties at T=37°C are most relevant to adhesion and removal properties. However, for the hydrogels of this invention, it has been found that the rheology properties are only at most moderately sensitive to temperature in the range of 25-37°C. Thus, for the purpose of this invention, it is convenient to specify the rheological properties at a temperature of 25°C. The adhesive has an elastic modulus (also referred to as storage modulus) at a temperature of 25°C abbreviated $G'_{25}$, a viscous modulus at a temperature of 25°C of $G''_{25}$, and the ratio of $G''_{25}$ / $G'_{25}$ at 25°C, referred to as tan $\delta_{25}$.

The elastic modulus (G') of the adhesive is a significant factor which influences the ability of hairs or fibers to be embedded within the hydrogel. Hydrogels with high values of G' are typically too stiff for hairs to be embedded to a significant degree and, for those hairs that do become embedded, too rigid to allow for facile removal without pain. The

$G'_{25}$ (1 rad/sec) of the present invention is selected to be less than 1000 Pa, preferably in the range of from 100Pa to 1000Pa, more preferably in the range of 100-700 Pa, and even most preferably in the range of 100-500 Pa.

The nature of the fibers also affects the ability of the fiber to penetrate into the hydrogel adhesive. For hair, it has been found that, in particular, the caliper, length, density, and curliness of the hair, which can vary with hair type, impacts the upper value of G' consistent with good penetration of hair into the hydrogel, allowing good gasketing and adhesion of the hydrogel to the underlying skin. It has been determined that the following equation relates the upper value of $G'_{25}$, (1 rad/s) in Pascals (Pa) consistent with good hair penetration to hair characteristics, based on hair from a referenced 1sqcm area.

$$G'_{25} \text{ (1 rad/sec)} < E/(W*C)$$

wherein :

    W = weight of hair per unit area of skin in $g/cm^2$
    C = hair curliness factor (ratio of length of stretched to unstretched hair)
    E = Numerical Factor for Hair Penetration in units of $Pa*g/cm^2$

We have found that for essentially complete penetration of hair the value for E is 24, preferably 19, even more preferably 9. Thus, for example, if the average value of W for a population type in a relevant 1sqcm area for adhesion is determined to be 0.02 $g/cm^2$, the average curliness factor for this hair based on microscopy analysis of the removed hairs is 1.2, then for E = 24, the value of $G'_{25}$, must be less than 1000 Pa to ensure good embedding of the hair into the hydrogel and thus good gasketing.

The fiber-embedding performance of the adhesive hydrogels herein can be measured by the degree of gasketing provided to the surface to which the hydrogels are applied; and a test method is described hereinafter. The hydrogels herein provide a degree of gasketing significantly higher compared to adhesive hydrogels having a elastic modulus above the present claimed range.

As mentioned above, the viscous modulus of the adhesive is also a significant factor which influence performance, and thus the $G''_{25}$ (1 rad/sec) of the present invention lie in the range of from 50 Pa to 1000 Pa, preferably from 100Pa to 700Pa.

Furthermore, the value of tan $\delta_{25}$ (1rad/sec) directly influences the cohesiveness of the adhesive hydrogels, thus the ability of the hydrogel to disengage from the surface, the fibers, without leaving residues.

The tan $\delta_{25}$ (1rad/sec) for the hydrogel herein should preferably be less than 0.65, preferably in the range from 0.15 to 0.55, more preferably in the range of 0.15-0.35.

Peel Force

It is a preferred characteristic of the hydrogels herein that their peel force, despite the fact the elastic modulus and tan $\delta$ have relatively low values (to ensure respectively good fiber embedding and a high level of cohesiveness), is maintained at an appropriate, value allowing to exhibit excellent adhesion performance on surfaces. In order to ensure the required adhesion initially, the adhesive has a peel strength on dry skin of from 0.1 N/cm to 5N/cm, preferably from 0.3 N/cm to 3N/cm most preferably 0.3 N/cm to 2 N/cm as determined according to the test method described herein. The peel force of the hydrogel herein, as measured on dry skin, should be in the range of from 0.1 to 5 N/cm, preferably 0.3 to 3N/cm.

Main Ingredients

According to the present invention the 3-dimensional matrix also referred to herein as a gel, comprises as an essential component a polymer which can be physically or chemically cross-linked. The polymer may be naturally or synthetically derived. The uncrosslinked polymer includes repeating units derived from vinyl alcohols, vinyl ethers and their copolymers, carboxy vinyl monomer, vinyl ester monomers, esters of carboxy vinyl monomers, vinyl amide monomers, hydroxy vinyl monomers, cationic vinyl monomers containing amines or quaternary groups, N-vinyl lactam monomer, polyethylene oxides, polyvinylpyrrolidon (PVP), acrylics such as hydroxyethylmethacrylate, methoxydiethoxyethyl methacrylate, hydroxydiethoxyethyl methacrylate, acrylic acid and acrylates and sulphonated polymers such as acrylamide sulphonated polymers, sulphopropylacrylates and mixtures thereof. Alternatively, the uncrosslinked polymer may be a homopolymer or copolymer of a polyvinyl ether, or a copolymer derived from half ester of maleic ester. Similarly any other compatible polymer monomer units may be used as copolymers such as for example polyvinyl alcohol and polyacrylic acid or ethylene and vinyl acetate.

As another alternative, the polymers may be block copolymer thermoplastic elastomers such as ABA block copolymers such as styrene-olefin-styrene block copolymers or ethylenepropylene block copolymers. More preferably such polymers include hydrogenated grade Styrol/Ethylene-Butylene/Styrol (SEBS), Styrene/Isoprene/Styrene (SIS), and Styrol/Ethylene-Propylene/Styrol (SEPS).

Particularly preferred polymers are acrylics, sulphonated polymers such as acrylamide sulphonated polymers, vinyl alcohols, vinyl pyrrolidine, polyethylene oxide and mixtures thereof.

The polymers herein can also be made of monomers which are selected from strong-acid monomers, weak-acid monomers, nonionic, cationic or zwitterionic monomers.

Strong-acid monomers is defined in relation to their pKa, which must be below 3. The pKa is measured by titration of the acid with strong base in aqueous solution according to methods well known in the art. The said strong-acid monomers are preferably selected from the group of olefically unsatured aliphatic or aromatic sulfonic acids such as 2-acrylamido-2-methylpropanesulfonic acid, 3-sulphopropyl (meth)acrylate, 2-sulfoethyl (meth)acrylate, vinylsulfonic acid, styrene sulfonic acid, allyl sulfonic acid, vinyl toluene sulfonic acid, methacrylic sulfonic acid and the like. Particularly referred strong-acid monomers are 2-acrylamido-2-methylpropanesulfonic acid, 3-sulfopropyl (meth)acrylate, 2-sulfoethyl (meth)acrylate.

Weak acid monomers are defined in relation to their pKa, which must be above 3. The said monomers are preferably selected from the group of olefinically unsaturated carboxylic acids and carboxylic acid anhydrides such as acrylic acid, methacyclic acid, maleic acid, itaconic acid, crotonic acid, ethacrylic acid, citroconic acid, fumaric acid, $\delta$-sterylacrylic acid and the like. Particularly preferred weak-acid monomers are acrylic acid and methacrylic acid.

Examples of nonionic monomers include N,N-dimethylacrylamide, acrylamide, N-isopropyl acrylamide, hydroxyethyl (meth)acrylate, hydroxypropyl (meth)acrylate, alkyl (meth)acrylates, N-vinyl pyrrolidone and the like. Examples of cationic monomers include N,N-dimethylaminoethyl (meth)acrylate, N,N-dimethylaminoethyl (meth)acrylamide and the respective quaternary salts and the like.

Humectant

The 3-dimensional adhesive matrix also comprises a humectant or mixture of humectants (also referred herein as a plastisizer), which is preferably a liquid at room temperature. The humectant is selected such that the monomer and polymer may be solubilized or dispersed within. For embodiments wherein irradiation cross linking is to be carried out, the humectant is desiderably irradiation cross linking compatible such that is does not significantly inhibit the irradiation cross linking process of the polymer. The components of the humectant mixture are preferably hydrophilic and miscible with water.

Suitable humectants include alcohols, polyhydric alcohols such as glycerol and sorbitol, and glycols and ether glycol such as mono- or diethers of polyalkylene glycol, mono- or diester polyalkylene glycols, polyethylene glycols (typically up to a molecular weight of about 600), glycolates, glycerol, sorbitan esters, esters of citric and tartaric acid, imidazoline derived amphoteric surfactants, lactams, amides, polyamides, quaternary ammonium compounds, esters such as phthalates, adipates, stearates, palmitates, sebacates, or myristates, glycerol esters, including mono/di/tri-glycerides, and combinations thereof. Particularly preferred are polyhydric alcohols, polyethylene glycol (with a molecular weight up to about 600), glycerol, sorbitol and mixtures thereof. Glycerol is especially preferred. The humectant comprises 5-80 wt% of the hydrogel.

An important function of the humectant is to reduce the water activity of the hydrogel to 0.35-0.95, preferably 0.4-0.85, most preferably from 0.45-0.75. Water activity is determined by measuring the equilibrium relative humidity above the hydrogel according to the method described hereinafter in the test methods section.

Suitable humectants include alcohols, polyhydric alcohols such as glycerol and sorbitol, and glycols and ether glycol such as mono- or diethers of polyalkylene glycol, mono- or diester polyalkylene glycols, polyethylene glycols (typically up to a molecular weight of about 600), glycolates, glycerol, sorbitan esters, esters of citric and tartaric acid, imidazoline derived amphoteric surfactants, lactams, amides, polyamides, quaternary ammonium compounds, esters such as phthalates, adipates, stearates, palmitates, sebacates, or myristates, glycerol esters, including mono/di/tri-glycerides, and combinations thereof. Particularly preferred are polyhydric alcohols, polyethylene glycol (with a molecular weight up to about 600), glycerol, sorbitol and mixtures thereof. Glycerol is especially preferred. The humectant comprises 5-80 wt% of the hydrogel.

An important function of the humectant is to reduce the water activity of the hydrogel to 0.35-0.95, preferably 0.4-0.85, most preferably from 0.45-0.75. Water activity is determined by measuring the equilibrium relative humidity above the hydrogel according to the method described hereinafter in the test methods section.

Polymerization conditions

According to the present invention the polymer component of the adhesive can be physically, chemically or ionically cross linked in order to form the 3 dimensional matrix. Physical cross linking refers to polymers having cross links which are not chemical covalent bonds but are of a physical nature such that for example there are three areas in the 3 dimensional matrix having high crystallinity or areas having a high glass transition temperature or areas having hydrophobic interactions. Chemical cross linking refers to polymers which are linked by chemical bonds. The polymer can be chemically cross linked by radiation techniques such as UV-, E beam-, gamma or micro-wave radiation or by co-polymerizing the monomers with a di/poly-functional crosslinker via the use e.g., of UV, thermal and/or redox polymerization initiators.

Suitable polyfunctional monomer crosslinkers include polyethyleneoxide d(meth)acrylates with varying PEG molecular weights, IRR280 (a PEG diacrylate available from UCB Chemical), trimethylolpropane ethoxylate tri(meth)acrylate with varying ethyleneoxide molecular weights, IRR210 (an alkoxylated tryacrilate: available from UCB Chemicals), trimethyolpropane tri(meth)acrylate, divinylbenzene, pentaerythritol triacrylate, pentaeythritol triallyl ether, triallyl amine, N,N-methylene-bis-acrylamide and other polyfunctional monomer crosslinkers known to the art. Preferred monomer crosslinkers include the polyfunctional diacrylates and triacrylates.

The monomers of the present invention are preferably polymerized via the use of a free radical polymerization initiator. Such free-radical polymerization initiators are well known in the art and can be one or more photoinitiator(s), thermal initiator(s), or redox initiator(s) and be present in quantities up to 5% by weight, preferably from 0.02 % to 2 %, more preferably from 0.02 % to 0.4 %. Photo initiators are preferred. Suitable photo initiators include type I-[]-hydroxyketones and benzyldimethyl-ketals e.g. Irgacure 651 (dimethoxybenzylphenone; available from Ciba Specialty Chemicals) which are believed, on irradiation with UV frequencies, to form benzoyl radicals that initiate polymerization. Particularly preferred photoinitiators include 2-hydroxy-2-methyl-propiophenone (available under the trade name of Darocur 1173 from Ciba Specialty Chemicals), I-hydroxycyclohexylphenylketone (available under the trade name Irgacure 184 from Ciba Specialty Chemicals) and 4-(2-hydroxyethoxy)phenyl-(2-hydroxy-2-methylpropyl) ketone (available under the trade name of Irgacure 2959 from Ciba Specialty Chemicals). Suitable thermal initiators include potassium persulfate and VA044 (available from Wako). Suitable redox initiators include the combination of hydrogen peroxide and ascorbic acid and sodium persulfate and ascorbic acid.

Chemical crosslinking can also be affected after polymerization by use of polyfunctional reagents capable of reacting with polymer functional groups such as ethyleneglycol diglycidyl ether, polyols such as glycerol, and other polyfunctional reagents known to the art.

Crosslinking can also be effected all or in part by ionic crosslinking wherein groups of opposite charge interact via ionic interactions. Suitable ionic crosslinking agents include those known to the art including polyvalent actions such as $Al^{+3}$ and $Ca^{+2}$, d/poly-amines, d/poly-quaternary ammonium compounds, including polymeric polyamines and poly-quaternary ammonium compounds known to the art.

In preparing adhesive compositions in accordance with the invention, the ingredients will usually be mixed to provide a reaction mixture in the form of an initial pre-gel aqueous based liquid formulation, and this is then converted into a gel by a free radical polymerization reaction as described above. This may be achieved for example using conventional thermal initiators and/or photoinitiators or by ionizing radiation. Photoinitiation is a preferred method and will usually be applied by subjecting the pre-gel reaction mixture containing an appropriate photoinitiation agent to UV light after it has been spread or coated as a layer on siliconised release paper or other solid or porous substrate. The incident UV intensity, at a wavelength in the range from 240 to 420nm is of sufficient intensity and exposure duration (e.g. 10-3000 mW/cm$^2$) to complete the polymerization in a reasonable time. To facilitate the process, it is often preferable to expose the reaction mixture to several UV irradiation sources, in sequence. The processing will generally be carried out in a controlled manner involving a precisely predetermined sequence of mixing and thermal treatment or history.

The total UV irradiation time should preferably be less than 300 seconds, more preferably less than 60 seconds, and even more preferably less than 10 seconds to form a gel with better than 95% conversion of the monomers, preferably more that 99.9% of monomers, even more preferably more than 99.99% of monomers. Those skilled in the art will appreciate that the extent of irradiation will be dependent on the thickness of the reaction mixture, reactivity and concentration of the monomers, concentration of photoinitiator, properties of the humectant, and nature of substrate on to which the reaction mixture is coated and the source of UV.

These timings are for high pressure mercury arc lamps as the source of UV operating at 200 W/cm. The peak intensity of UV reaching the surface of the substrate is approximately 1000nvW/cm$^2$. For a given lamp, the UV intensity is a function of the operating power and distance of the reaction mixture from the UV source. Also, a high-pass UV filter can be employed to minimize exposure to UV intensities of very-low wavelength.

In order to minimize and preferably eliminate the presence of any residual monomers it is important to ensure that the reaction is complete. This is dependent upon a number of factors such as the substrate onto which the adhesive is applied, the type and intensity of the ultra violet light and the number of ultra violet light passes.

Optional Ingredients

Common additives known in the art such as polymerization inhibitors, chain transfer agents, surfactants, soluble or dispersible polymers, buffers, preservatives, antioxidants, pgments, mineral fillers, and the like, and mixtures thereof, may also be comprised within the adhesive composition in quantities up to 10% by weight each rcspoectively. Preferably, the hydrogels herein should contain no salt or minimum levels, below 1% by weight, preferably below 0.5% by weight.

pH

The pH of the hydrogel composition herein is in the range of from 3 to 6, more preferably 3 to 5.5, most preferably from 3.5 to 5.5, which represents values perfectly compatible with the pH of mammalian skin.
This pH range is directly achievable by the compositions herein, without, without the use of any additional buffering agent.
The conditions of measure of the pH are described hereinafter in the test methods section.

Surface Care Articles

In a second embodiment herein, the present invention is directed to Surface Care Articles.
For the purpose of the present invention, Surface Care Articles means articles, disposable or removable, which are designed to be temporary applied to a surface in order to achieve a function directed to the treatment of said surface, and thus require temporary adhesion to said surface.
The surfaces to which the articles are particularly adapted are fiber-populated surface which includes any durable fabric used in garment upholstery, carpet as well as disposable fabrics such as nonwovens.
The treatment function to be achieved by the present invention articles can range from the permanent or temporary application of textile finishing, the cleaning of garment, upholstery or carpets, to the application on the durable or disposable fabrics, of substances such as perfumes or other actives designed to emanate from the textile.
An example of a surface-care article according to the present invention is a carpet dusting implement, containing an adhesive hydrogel according to the present invention, applied to carpets as a substitute or a complement to vacuum cleaning; the hydrogel may be present as a roll of disposable wipes rolled onto a mop, used as a carpet cleaning implement; other application of such a mop execution can be envisaged, such a dusting / cleaning of upholstery, curtains and garments.

Test Methods

*1. Rheology*

The rheology of hydrogels is measured at 25°C using a RHEOMETRICS SR 5000 oscillatory rheometer or the equivalent. A sample of thickness of approximately 1mm and diameter of 25 mm is placed between two insulated Parallel Plates of 25mm diameter, controlled at a temperature of approximately 25°C using a Peltier system or equivalent. A Dynamic Frequency Sweep is performed on the hydrogel in either stress or strain mode at an applied strain within the linear elastic response of the hydrogel (e.g., up to a strain of about 10%), with measurements at discrete frequency values between 0.1 and 100 rad/sec. Results are quoted as G', G" and tan delta at frequency values of 1.0 and 100 rad/sec. The hydrogel is aged at least 24 hours before measurement. The average of at least three determinations are reported.

*2. Peel force on PET*

Peel force to remove hydrogel from poly(ethylene teraphthalate) (PET) film is measured using a suitable tensile tester, for example an Instron Model 6021, equipped with a 10N load cell and attachment for a rigid lower plate, e.g. steel, oriented along the direction of cross-head movement. Freshly produced hydrogel is stored in a closed aluminium bag or similar for at least 12 to 24 hours at room temperature before measuring. A defect free sample of at least 10cm in length is cut from the hydrogel sample. A piece of double sided adhesive, for example type 1524 from 3M Italia S.p.A, I-20090 Segrate, Italy, at least 130mm long and 25.4mm wide is stuck to the back side of the hydrogel laminate. The hydrogel is cut along the tape's outer edges. The second liner is removed from the tape and it is stuck on the rigid base plate. A strip of standard PET of 23μ thickness and no corona treatment, is cut to about 300mm x 40mm. Suitable material would include "Cavilen-Forex" from Effegidi S.p.A, Via Provinciale per Sacca 55, I-43052 Colorno, Italy. The release liner is removed from the hydrogel and the bottom end fixed to the rigid plate by regular tape. The standard substrate is then applied onto the body adhesive using a hand roller once forward and once backward at a speed of 1000 to 5000 mm/min. The roller is 13cm in diameter, 4.5cm wide and has a mass of 5Kg. It is covered in rubber of 0.5mm thickness. The measurement is preferably performed within 10 minutes of application of the substrate.

The free end of the standard substrate is doubled back at an angle of 180 degrees and the rigid plate is clamped in the lower clamp of the tensile tester. The free end of the standard substrate is fixed in the upper clamp of the tensile tester. The peel test is performed at a speed of 1000mm/min. The initial 20mm of peel is disregarded and the average force over the remaining length is quoted as the peel force in N/cm. The average of triplicate measurements is reported.

3. *Fiber Embedding Test*

A sample of hydrogel adhesive on a liquid-impermeable substrate with lateral dimensions greater than 35 mm is used for this test. Also used for this test is a high-loft hydrophobic non-woven with basis weight of approximately 35 gsm (for example, Sandler 4378 Sawabond non-woven or equivalent) which has lateral dimensions equal to or greater than that of the hydrogel. Prior to use, the back side of this non-woven (the non-high-loft side) is covered with (i) a double sided adhesive (for example type 1524 from 3M Italia S.p.A, I-20090 Segrate, Italy) and (ii) a piece of standard PET of 23μ thickness and no corona treatment, (for example "Cavilen-Forex" from Effegidi S.p.A, Via Provinciale per Sacca 55, I-43052 Colorno, Italy). The hydrogel sample is applied co-facially on top of the nonwoven with the adhesive side of the hydrogel in contact with the high-loft side. An approximately 35 mm diameter circular piece of the hydrogel/non-woven sandwich structure is cut from the larger piece using a circular punch or equivalent of inner diameter of approximately 35 mm and weighed to an accuracy of at least 0.001 g ($W_{35}$). A compression weight roller with a diameter of approximately 13cm and a mass of approximately 5Kg, which is covered in rubber of approximately 0.5mm thickness, is used to compress the resultant 35 mm diameter piece of hydrogel/non-woven sandwich structure by rolling the compression weight on top of the piece first forward and then backward. The compressed circular piece of hydrogel/non-woven sandwich is positioned with the non-woven side down in a dry container and covered with a cylindrical stainless steel weight of diameter approximately 50 mm and weight of approximately 240 gm. To this container is then added a sufficient volume of 0.9% saline solution, containing approximately 0.01 wt% of Indigo Carmine Blue Dye, to completely cover the sandwich structure: After an immersion time of approximately 60 minutes, the test sample is removed from the saline and the outer surfaces are blotted dry. An approximately 25 mm diameter piece is cut from the center of the 35 mm diameter test sample using a circular punch or equivalent having an inner diameter of approximately 25 mm and weighed to an accuracy of at least 0.001 g ($W_{25}$). By cutting away the outer 5 mm wide ring of the test sample, hydrogel that is swollen solely by diffusive contact with the saline is removed. This central 25 mm diameter piece is also visually checked for the appearance of blue color. The weight gain of the central 25 mm diameter inner section of the hydrogel in percent as a result of immersion of the larger 35 mm test piece in saline is calculated using the following equation:

$$\text{Weight Gain (\%)} = 100 * \{(W_f - W_i)/W_i\} / F$$

where,

$$F = \{ W_{35} - (BW_{nws} * A_{35}) - (BW_{substrate} * A_{35})\}/W_{35}$$

and

$W_i = W_{35}*A_{25}/A_{35}$
$W_f = W_{25}$
$W_{35}$ = Dry weight of 35 mm circular piece of hydrogel/non-woven in grams
$A_{35}$ = Area of 35 mm circular piece of hydrogel/non-woven in cm$^2$
$A_{25}$ = Area of 25 mm circular piece of hydrogel/non-woven in cm$^2$
$W_{25}$ = Weight of 25 mm inner circle of hydrogel/non-woven after Immersion in grams
$BW_{nws}$ = Weight per unit area of non-woven structure in units of g/cm$^2$
$BW_{substrate}$ = Weight per unit area of hydrogel substrate in units of g/cm$^2$

The average of at least triplicate determinations are used to calculate the Percentage Weight Gain.

For good fiber embedding into the hydrogel, there are relatively few capillary paths for the saline solution to wick between the hydrogel and the hydrophobic backing of the non-woven and thus there is a low percentage weight gain (e.g., <5%) for the hydrogel in the 25 mm inner circle of hydrogel. There is also a minimal appearance of blue color in this central section of hydrogel. For poor fiber embedding, there are many capillary pathways and thus there is a greater weight gain (e.g., >5%). There is also a significant appearance of blue color in this central section of hydrogel.

**Claims**

1.  Use for adhesion on fiber populated surfaces of a hydrogel adhesive comprising 10-60 wt% of a cross-linked hydrophilic polymer; 5-80 wt% of a water-soluble nonionic humectant, and 10-85 wt% water, said adhesive having a elastic modulus at a temperature of 25°C, $G'_{25}$ selected such that $G'_{25}$ (Irad/see) is less than 1000Pa:

2.  Use according to Claim 1 wherein $G'_{25}$ (Irad/sec) is in the range of 100 to 700Pa.

3.  Use according to Claims 1-2 wherein the viscous modulus of said adhesive is selected such that $G''_{25}$ (1rad/sec) is in the range of 50 to 1000 Pa preferably 100 to 700Pa; the ratio $G''_{25}$ (1rad/sec)/$G'_{25}$, (1rad/sec) is in the range of 0.15 to 0.65, preferably 0.15 to 0.55, most preferably 0.15 to 0.35 and the peel force on dry skin is in the range 0.3 to 5.0N/cm, preferably 0.3 to 2.0 N/cm.

4.  Use according to Claims 1-3 wherein said cross-linked hydrophilic polymer contains monomer units selected from strong acid monomers having a pKa below 3 and weak-acid monomers having a pKa above 3, as well as nonionic, cationic or zwitterionic monomers.

5.  Use according to Claims 1-4 wherein said water-soluble non-ionic humectant is selected from polyhydric alcohols and is, preferably glycerol.

6.  A surface care article capable of adhering to fiber-populated surfaces such as fabrics, wherein said article comprises a hydrogel adhesive for adhesion on hair or fiber populated surfaces of a hydrogel adhesive comprising 10-60 wt% of a cross-linked hydrophilic polymer: 5-80 wt% of a water-soluble nonionic humectant, and 10-85 wt% water, said adhesive having a elastic modulus at a temperature of 25°C, $G'_{25}$ selected such that $G'_{25}$ (1 rad/sec) is less than 1000Pa.

7.  A surface care article according to Claim 13 wherein $G'_{25}$ (1 rad/sec) is in the range of 100 to 700Pa.

**Patentansprüche**

1.  Verwendung eines Hydrogel-Klebstoffes, der zu 10-60 Gew.-% ein vernetztes hydrophiles Polymer; zu 5-80 Gew.-% ein wasserlösliches nichtionisches Feuchthaltemittel und zu 10-85 Gew.-% Wasser aufweist, zur Anhaftung auf faserbesetzten Oberflächen, wobei der Klebstoff einen Elastizitätsmodul bei einer Temperatur von 25 °C, $G'_{25}$, hat, der so ausgewählt ist, dass $G'_{25}$ (1rad/s) weniger als 1000 Pa beträgt:

2.  Verwendung nach Anspruch 1, wobei $G'_{25}$ (1 rad/s) im Bereich von 100 bis 700 Pa liegt.

3.  Verwendung nach den Ansprüchen 1-2, wobei der Viskositätsmodul des Klebstoffes so ausgewählt ist, dass $G''_{25}$ (1rad/s) im Bereich von 50 bis 1000 Pa, vorzugsweise 100 bis 700 Pa liegt; das Verhältnis $G''_{25}$ (1rad/s)/$G'_{25}$ (1 rad/s) im Bereich von 0,15 bis 0,65, vorzugsweise 0,15 bis 0,55, am meisten bevorzugt 0,15 bis 0,35 liegt und die Schälkraft auf trockener Haut im Bereich 0,3 bis 5,0 N/cm, vorzugsweise 0,3 bis 2,0 N/cm liegt.

4.  Verwendung nach Ansprüchen 1-3, wobei das vernetzte hydrophile Polymer Monomereinheiten, ausgewählt aus Monomeren starker Säure mit einem pKa unter 3 und Monomeren schwacher Säure mit einem pKa über 3 sowie nichtionischen, kationischen oder zwitterionischen Monomeren, enthält.

5.  Verwendung nach den Ansprüchen 1-4, wobei das wasserlösliche nichtionische Feuchthaltemittel aus Polyolen ausgewählt ist und vorzugsweise Glycerol ist.

6.  Oberflächenpflegeartikel, der in der Lage ist, an faserbesetzten Oberflächen, wie textilen Stoffen, zu haften, wobei der Artikel einen Hydrogel-Klebstoff zum Anhaften eines Hydrogel-Klebstoffs, der zu 10-60 Gew.-% ein vernetztes hydrophiles Polymer, zu 5-80 Gew.-% ein wasserlösliches nichtionisches Feuchthaltemittel und zu 10-85 Gew.-% Wasser aufweist, auf haar- oder faserbesetzten Oberflächen umfasst, wobei der Klebstoff einen Elastizitätsmodul bei einer Temperatur von 25°C, G'$_{25}$, hat, der so ausgewählt ist, dass G'$_{25}$ (1 rad/s) weniger als 1000 Pa beträgt.

**7.** Oberflächenpflegeartikel nach Anspruch 6, wobei G'$_{25}$ (1rad/s) im Bereich von 100 bis 700 Pa liegt.

**Revendications**

**1.** Utilisation pour adhésion sur des surfaces garnies de fibres d'un hydrogel adhésif comprenant de 10 à 60 % en poids d'un polymère hydrophile réticulé ; de 5 à 80 % en poids d'un humidifiant non ionique hydrosoluble, et de 10 à 85 % en poids d'eau, ledit adhésif ayant un module d'élasticité à une température de 25 °C, G'$_{25}$ choisi de telle sorte que G'$_{25}$ (1 rad/sec) est inférieur à 1000 Pa.

**2.** Utilisation selon la revendication 1, dans laquelle G'$_{25}$ (1 rad/sec) est dans la gamme allant de 100 à 700 Pa.

**3.** Utilisation selon les revendications 1 à 2, dans lesquelles le module de viscosité dudit adhésif est choisi de telle sorte que G"$_{25}$ (1 rad/sec) soit dans la gamme allant de 50 à 1000 Pa, de préférence de 100 à 700 Pa ; le rapport G"$_{25}$ (1 rad/sec)/G'$_{25}$ (1 rad/sec) soit dans la gamme allant de 0,15 à 0,65, de préférence de 0,15 à 0,55, le plus préférablement de 0,15 à 0,35 et la force de pelage sur peau sèche soit dans la gamme allant de 0,3 à 5,0 N/cm, de préférence de 0,3 à 2,0 N/cm.

**4.** Utilisation selon les revendications 1 à 3, dans lesquelles ledit polymère hydrophile réticulé contient des motifs monomères choisis parmi les monomères possédant une fonction acide fort ayant un pKa inférieur à 3 et des monomères possédant une fonction acide faible ayant un pKa supérieur à 3, ainsi que des monomères non ioniques, cationiques ou zwittérioniques.

**5.** Utilisation selon les revendications 1 à 4, dans laquelle ledit humidifiant non ionique hydrosoluble est choisi parmi les alcools polyhydriques et est, de préférence, du glycérol.

**6.** Article pour l'entretien des surfaces susceptible d'adhérer aux surfaces garnies de fibres telles que des tissus, où ledit article comprend un adhésif hydrogel pour l'adhésion sur des cheveux ou des surfaces garnies de fibres d'un adhésif hydrogel comprenant de 10 à 60 % en poids d'un polymère hydrophile réticulé : de 5 à 80 % en poids d'un humidifiant non ionique hydrosoluble, et de 10 à 85 % en poids d'eau, ledit adhésif ayant un module élastique à une température de 25 °C, G'$_{25}$ choisi de telle sorte que G'$_{25}$ (1 rad/sec) est inférieur à 1000 Pa.

**7.** Article pour l'entretien des surfaces selon la revendication 6, dans lequel G'$_{25}$ (1 rad/sec) est dans la gamme allant de 100 à 700 Pa.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1025823 A
- EP 1025866 A
- WO 0007636 A
- EP 1026219 A
- US 2002026005 A